# EUROPEAN PATENT APPLICATION

(11) **EP 4 102 796 A1**
(43) Date of publication of application: **14.12.2022**
(21) Application number: 21178066.3
(22) Date of filing: 07.06.2021
(51) Int. Cl.: H04L 29/06

(54) **METHOD, COMPUTER PROGRAM PRODUCT AND PROCESSING CIRCUITRY FOR MAKING MEDICAL DATA AVAILABLE TO THIRD PARTIES**

(71) Applicant: Addi Medical AB, 182 33 Danderyd (SE)
(72) Inventor: STRIHAGEN, Björn, 187 33 TÄBY (SE); SELLBERG, Nina, 114 45 STOCKHOLM (SE)
(74) Representative: Brann AB

(57) **Abstract**

Medical data are made available to third parties via a server (100). The server (100) has a first interface (110) through which a digital storage agreement (R[auth]) is obtained from a terminal (UT). The digital storage agreement (R[auth]) authorizes storage of medical data (PD_{ID}) relating to a user of the terminal (UT) in a central database (140) connected to the server (100). In response to the digital storage agreement (R[auth]), a second interface (120) of the server (100) sends a first data request (RQ1) to a primary server (JS). The first data request (RQ1) causes the primary server (JS) to forward medical data (PO_{ID}) relating to the user to the second interface (120). The server (100) stores the obtained medical data (PO_{ID}) in the central database (140). A third interface (130) receives a data enquiry (ENQ) from a third party (DU) with a request for the medical data (PO_{ID}) relating to the user stored in the central database (140). In response thereto, the server (100) checks if the user has authorized (ACC) sharing the medical data (PD_{ID}) requested in the data enquiry (ENQ) with the third party (DU). Only if the user has authorized (ACC) such sharing, the server (100) forwards a copy of the medical data (PD_{ID}) requested in the data enquiry (ENQ) to the third party (DU).

## Description

### TECHNICAL FIELD

The invention relates generally to authorized and trustworthy storage and handling of sensitive data. In particular, the present invention concerns a computer-implemented method for making medical data available to third parties, and a server configured to implement such a method. The invention also relates to a computer program product and a non-volatile data carrier.

### BACKGROUND

In medical science and businesses, there is a general demand for patient-related data in order to perform research and develop new and improved drugs. In other words, there is a need to access, combine and process personal health data outside of the environment where such data is created, stored and used, namely in the health care services. Medical data originating from one or more health care providers form a very valuable basis for an evidence-based process treatment, where the data fulfils the scientific requirements of accuracy and traceability. It is further advantageous if the patients themselves may add pieces of information in such a process via an auxiliary input channel.

The need to access, combine and process personal health data outside the original source is driven by many factors. First, there is an obvious need for the patient himself/herself to perform self-treatment after, or in combination with, the care provided by a health care provider. Second, there is a vast number of research projects that are fully dependent on personal health data to make progress. This research includes academic research, the pharma industry as well as other independent actors.

WO 2018/046495 discloses a method in a healthcare monitoring system for anonymous communication of patient data associated with a patient from an electronic user device, using a patient application implemented in the electronic user device, to a host server, using a host application implemented in the host server, via a wireless network, and identification of the patient associated with the patient data after the patient data is received in the host server. The document further provides a corresponding system, computer program and non-volatile data carrier containing the computer program.

Thus, secure communication of patient data is enabled. However, the problem of making collections of medical data available to external parties, e.g. in academia and the pharma industry, remains to be solved. Namely, legislation and various regulations often prevent the data from a health care provider to be shared with external parties, and indeed even other health care providers.

Today, there are technical solutions which legally allow patients to view at least selected parts of the medical data relating to themselves. Primarily for personal-integrity reasons these solutions are designed not allow or enable any external parties to gain direct access to the medical data. This complicates the sharing of medical data with third parties.

### SUMMARY

It is therefore an object of the present invention to offer a solution for making medical data available to third parties in a convenient and straightforward manner, and at the same time fulfil all legal, regulatory and ethical conditions relating such sharing of data.

According to one aspect of the invention, this object is achieved by a method for making medical data available to third parties. The method is performed in at least one processor and involves obtaining, via a first interface, a digital storage agreement from a terminal, e.g. a smartphone, a laptop or a personal computer. The digital storage agreement authorizes storage of medical data in a central database, which medical data relates to a user of the terminal. In other words, a patient authorizes storage of his/her personal medical through an authorization process implemented in a user terminal. In response to the digital storage agreement, the method involves sending, via a second interface, a first data request to a primary server, for instance controlled by a health care provider. The first data request is configured to cause the primary server to forward medical data relating the user from the primary server to the central database. The method further involves obtaining medical data relating to the user via the second interface, and storing the obtained medical data in the central database. Via a third interface, a data enquiry is received from a third party, which data enquiry encompasses a request for the medical data relating to the user and which medical data are stored in the central database. In response to the data enquiry, the method involves checking if the user has authorized sharing the medical data requested in the data enquiry with the third party. If, and only if, the user has authorized such sharing, the method involves forwarding a copy of the medical data requested in the data enquiry to the third party via the third interface.

This method is advantageous because it enables authorized external parties convenient and low-latency access to personal medical data from large numbers of individuals for clearly specified purposes, such as fundamental research, applied research, and drug development, without violating any legal or regulatory conditions.

According to one embodiment of this aspect of the invention, the method involves receiving, via the first interface, a digital sharing authorization from the terminal, which digital sharing authorization is configured to authorize the sharing of the medical data requested in the data enquiry with the third party. Further, the method involves storing the digital sharing authorization in a contract database. Thus, it is straightforward to check whether a user has authorized a particular sharing of data whenever a data enquiry is received from a third party.

Preferably, the checking if the user has authorized sharing the medical data requested in the data enquiry with the third party involves the following: searching the contract database for the digital sharing authorization; and allowing forwarding of the copy of the medical data requested in the data enquiry to the third party via the third interface exclusively if the digital sharing authorization is found in the contract database. Thereby, the checking can be performed efficiently and automatically.

According to another embodiment of this aspect of the invention, the digital sharing authorization defines either a specific subset of the medical data relating to the user stored in the central database, or a complete amount of this data. This is advantageous because it allows each patient to tailor which data that can be shared to whom and for what purposes.

According to yet another embodiment of this aspect of the invention, the digital sharing authorization has a time limit after which it expires and ceases to be valid. This provides additional flexibility in terms of the circumstances under which data can be shared.

Analogous to the above, according to still another embodiment of this aspect of the invention, the digital storage agreement either defines a specific subset of the medical data user stored in a primary database controlled by the primary server, or a complete amount of this data. Thereby, the patient may tailor which data that that he/she accepts to be forwarded from the primary server to the central server.

Preferably, the digital sharing authorization may also have a time limit after which it expires and ceases to be valid. Hence, the medical data do not risk being permanently stored in the central database.

According to another aspect of the invention, the object is achieved by a computer program product loadable into a non-volatile data carrier being communicatively connected to at least one processor. The computer program product contains software configured to, when the computer program product is run on the at least one processing circuitry, cause the at least one processing circuitry to effect the above-described method. The advantages of this computer program product and non-volatile data carrier are apparent from the discussion above with reference to the proposed method.

According to yet another aspect of the invention, the above object is achieved by a server for making medical data available to third parties. The server contains first, second and third interfaces, and is communicatively connected to a central database.

The first interface is configured to obtain a digital storage agreement from a terminal, which digital storage agreement authorizes storage of medical data relating to a user of the terminal in the central database. The second interface is configured to send, in response to the digital storage agreement, a first data request to a primary server, e.g. controlled by a health care provider, which first data request is configured to cause the primary server to forward medical data relating to the user to the second interface. Thus, the second interface is also configured obtain medical data relating to the user from the primary server. The server is configured to store the obtained medical data in the central database. The third interface is configured to receive a data enquiry from a third party, which data enquiry encompasses a request for the medical data relating to the user stored in the central database. In response to the data enquiry, the server is further configured to check if the user has authorized sharing the medical data requested in the data enquiry with the third party. Only if the user has authorized such sharing, the server is configured to forward a copy of the medical data requested in the data enquiry to the third party. The advantages of this server are apparent from the discussion above with reference to the proposed method.

Further advantages, beneficial features and applications of the present invention will be apparent from the following description and the dependent claims.

### BRIEF DESCRIPTION OF THE DRAWINGS

The invention is now to be explained more closely by means of preferred embodiments, which are disclosed as examples, and with reference to the attached drawings.
- Figure 1: shows a block diagram of a system according to one embodiment of the invention;
- Figure 2: illustrates a procedure according to which medical data are made available to a third party according to one embodiment of the invention; and
- Figure 3: illustrates, by means of a flow diagram, the general method according to the invention for making medical data available to third parties.

### DETAILED DESCRIPTION

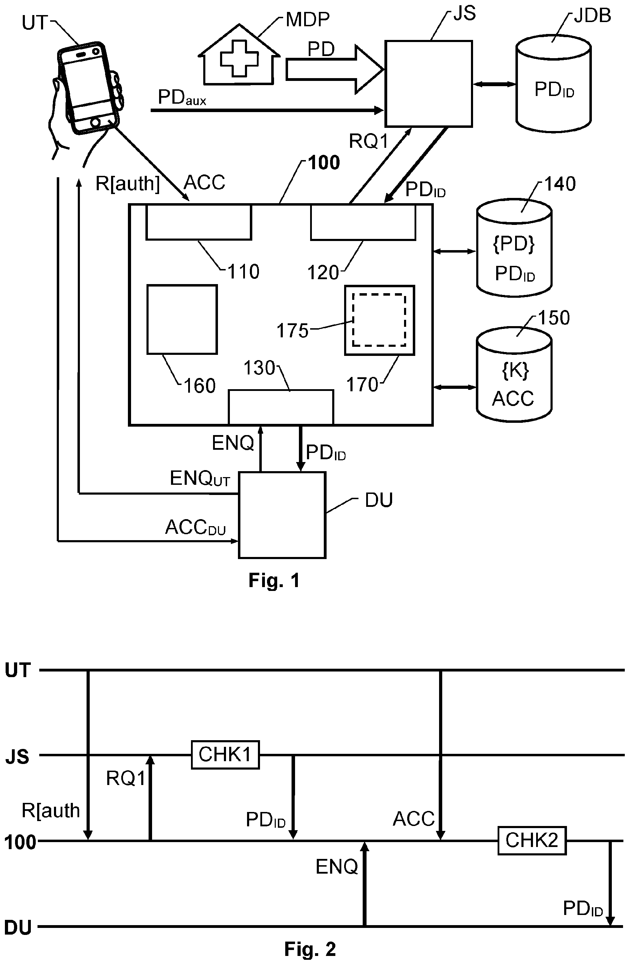

Figure 1 shows a block diagram of a system that includes a server 100 according to one embodiment of the invention. Figure 2 illustrates a procedure according to which medical data are made available to third parties via the server 100. In the below description, we refer to Figures 1 and 2 in parallel.

The server 100 contains first, second and third interfaces 110, 120 and 130 respectively, and is communicatively connected to a central database 140.

The first interface 110 is configured to obtain a digital storage agreement R[auth] from a terminal UT, e.g. a smartphone, a tablet, a laptop or a personal computer. The digital storage agreement R[auth] authorizes storage of medical data PD_{ID} relating to a user of the terminal UT in the central database 140, for example based on a so-called strong authentication process involving a unique access key and exchange of a randomized numeric code between the terminal UT and the server 100. Preferably, the digital storage agreement R[auth] is generated by means of a dedicated software, e.g. a so-called app, installed in the terminal UT, which software is arranged to establish a secure connection to the first interface 110 of the server 100, for example over the Internet. Such a dedicated software in the terminal UT facilitates certifying that the digital storage agreement R[auth] indeed originates from an authorized person, for example by requesting digital signatures, using a chain of trust, forwarding the credentials of the person logged into the server 100, or by requiring that the user of the terminal UT re-authenticates himself/herself.

The digital storage agreement R[auth] defines an identity of the user, i.e. a subject to whom a specified amount of data PD_{ID} relates. The data PD_{ID}, in turn, are presumed to be stored in a primary database JDB, which is controlled by the primary server JS. Typically, the data PD_{ID} form part of a medical journal created by a health care provider MDP for the subject. However, the invention does not preclude that there is also an auxiliary channel for entering additional data PDₐᵤₓ, for example from the terminal UT, which auxiliary channel allows a patient to provide information supplementing the medical data entered by the health care provider MDP.

For example, a set of digital storage agreements R[auth] that pertain to a given health care provider may be organized in a common collection {PD} in the database 140.

Preferably, the digital storage agreement R[auth] defines either a subset, or a complete amount of the medical data PD_{ID} relating to the user that are stored in the primary database JDB and being controlled by the primary server JS. The digital storage agreement R[auth] may further define a set of purposes for which the medical data PD_{ID} are allowed to be used and/or a time limit after which the digital storage agreement R[auth] expires and ceases to be valid. Thus, when the time limit has been passed, the medical data PD_{ID} are deleted from the central database 140.

In response to the digital storage agreement R[auth], the second interface 120 is configured to send a first data request RQ1 to the primary server JS. The first data request RQ1 is configured to cause the primary server JS to forward medical data PD_{ID} relating to the user to the second interface 120. Here, exclusively medical data PD_{ID} fulfilling the conditions of the digital storage agreement R[auth] are forwarded from the primary server JS. This is verified by means of a first checking procedure CHK1 performed in the primary server JS. Provided that the first checking procedure CHK1 is passed, and the medical data PD_{ID} are forwarded from the primary server JS, the medical data PD_{ID} relating to the user from the primary server JS are obtained in the server 100 via the second interface 120.

The server 100 is then configured to store the obtained medical data PD_{ID} in the central database 140, so that the medical data PD_{ID} may be held available by the server 100 at a later point in time.

The third interface 130 is configured to receive a data enquiry ENQ from a third party DU, or a data user, for example in the form of a research institute, a university or a pharmaceutical company.

The data enquiry encompasses a request for the medical data PD_{ID} relating to the user, which medical data PD_{ID} are stored in the central database 140. Naturally, in practice, the data enquiry ENQ also encompasses medical data relating to many other patients, perhaps in the order of thousands, tens or hundreds of thousands. For simplicity, however, in this description, we only discuss the medical data PD_{ID} relating to a single individual.

In response to the data enquiry ENQ, the server 100 is further configured to perform a second checking procedure CHK2 in which it is checked if the user has authorized sharing the medical data PD_{ID} requested in the data enquiry ENQ with the third party DU. If and only if the user has authorized such sharing, the server 100 is further configured to forward a copy of the medical data PD_{ID} requested in the data enquiry ENQ to the third party DU via the third interface 130.

According to one embodiment of the invention, the server 100 is configured to receive a digital sharing authorization ACC from the terminal UT via the first interface 110. The digital sharing authorization ACC is configured to authorize the sharing of the medical data PD_{ID} requested in the data enquiry ENQ with the third party DU.

According to embodiments of the invention, the digital sharing authorization ACC may define either a subset, or a complete amount of the medical data PD_{ID} relating to the user stored in the central database 140. Additionally, or alternatively, the digital sharing authorization ACC may have a time limit after which it expires and ceases to be valid. In other words, after the time limit, none of the medical data PD_{ID} relating to the user will be shared with any third parties DU.

Obviously, it is difficult for the user to know in advance whether a particular third party DU will issue a data enquiry ENQ, and if so, which type of medical data that will be encompassed by the data enquiry ENQ. Therefore, in connection with, and preferably prior to, issuing the data enquiry ENQ, the third part DU may send a corresponding enquiry ENQ_{UT} to the user, for example via the above-mentioned software in the terminal UT. Consequently, in response to the data enquiry ENQ, the user may send a digital sharing authorization ACC through which the user authorizes sharing the medical data PD_{ID} requested in the data enquiry ENQ with the third party DU.

Moreover, it is convenient if the terminal UT sends an equivalent message ACC_{DU} to the third part DU in parallel with the digital sharing authorization ACC, which equivalent message ACC_{DU} mirrors the authorization sent to the server 100. Namely, thereby the third part DU gains heads up information about what medical data can be expected to be held available via the server 100.

According to one embodiment of the invention, the server is configured to store the digital sharing authorization ACC in a contract database 150. For instance, a set of digital sharing authorizations ACC pertaining to a given third party DU may be organized in a common collection {K} in the contract database 150.

According to one embodiment of the invention, the server 100 is configured to perform the second checking procedure CHK2 if the user has authorized sharing the medical data PD_{ID} requested in the data enquiry ENQ with the third party DU as follows:
(i) searching the contract database 150 for the digital sharing authorization ACC; and
(ii) allowing forwarding of the copy of the medical data PD_{ID} requested in the data enquiry ENQ to the third party DU via the third interface 130 exclusively if the digital sharing authorization ACC is found in the contract database 150.

Legally, this is equated to sharing the medical data PD_{ID} with the user/patient via a first proxy issued by the user to the server 100, and a second proxy issued by the server 100 to the third party DU in the user's name with respect of the medical data PD_{ID}. Hence, the server 100 implements a two-step proxy service for all the users/patients who have authorized sharing their medical data PD_{ID} with one or more third parties DU. This, in turn, renders the server 100 a highly efficient tool for making various collections of medical data available to external parties in the form of academia and pharma industry.

In Figure 1, it is presumed that the central server 100 contains at least one processor, here symbolized by 160, which is communicatively connected to a non-volatile data carrier 170, which may either be included in the central server 100, or be located in a unit external thereto. The non-volatile data carrier 170 stores a computer program product 175 containing software configured to, when the computer program product 735 is run on the at least one processor 160, cause the at least one processor 160 to carry out the above-described procedure.

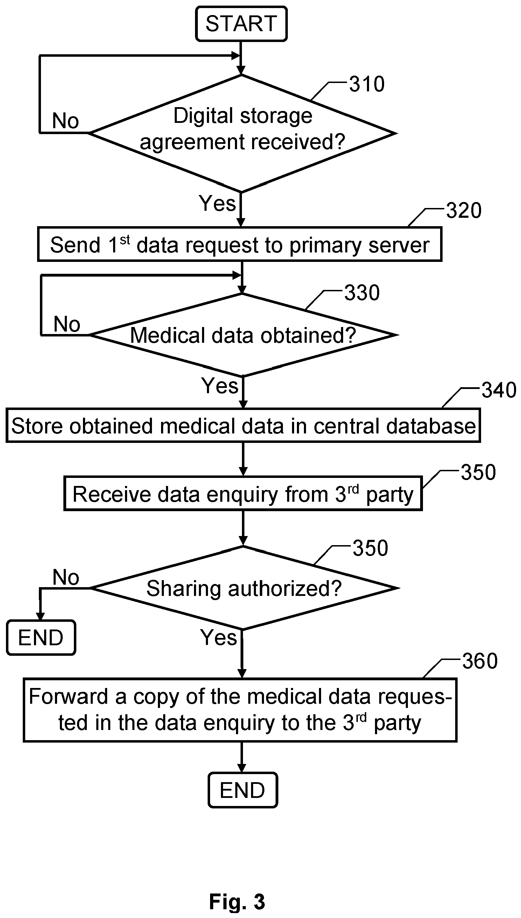

In order to sum up, and with reference to the flow diagram in Figure 3, we will now describe the general method according to the invention for making medical data available to third parties, which method is performed in at least one processor of at least one server, e.g. implementing a so-called cloud service.

A first step 310 checks if a digital storage agreement R[auth] has been received from a terminal UT via a first interface 110. The digital storage agreement R[auth] authorizes storage of medical data PD_{ID} in a central database 140, which medical data relates to a user of the terminal UT. If such a digital storage agreement R[auth] has been received, a step 320 follows; and otherwise, the procedure loops back and stays in step 310.

In step 320, a first data request is sent to a primary server JS via a second interface 120. The first data request RQ1 is configured to cause the primary server JS to forward medical data PD_{ID} relating the user from the primary server JS to the central database 140.

Then, a step 330 checks if medical data PD_{ID} relating to the user have been obtained via the second interface 120; and if so, a step 340 follows. Otherwise, the procedure loops back and stays in step 330. In step 340, the obtained medical data PD_{ID} are stored in the central database 140.

Thereafter, the procedure pauses until a data enquiry ENQ from a third party DU. Consequently, if no such data enquiry ENQ is received, the procedure stops in step 340.

Here, however, in a step 350, we assume that a data enquiry ENQ from a third party DU is received via a third interface 130. The data enquiry ENQ encompasses a request for the medical data PD_{ID} relating to the user and which medical data PD_{ID} are stored in the central database 140.

Subsequently, a step 350 checks if the user has authorized sharing the medical data PD_{ID} requested in the data enquiry ENQ with the third party DU. Only if the user has authorized such sharing a step 360 follows. This means that, if the user has *not* authorized such sharing, the procedure ends after step 350. It is worth noticing that the user authorization may contain conditions not only in terms of which data can be shared, however also for what purposes and until which latest point in time.

In step 360, a copy of the medical data PD_{ID} requested in the data enquiry ENQ is forwarded to the third party DU via the third interface 130.

All of the process steps, as well as any sub-sequence of steps, described with reference to Figure 3 above may be controlled by means of at least one programmed processor. Moreover, although the embodiments of the invention described above with reference to the drawings comprise processor and processes performed in at least one processor, the invention thus also extends to computer programs, particularly computer programs on or in a carrier, adapted for putting the invention into practice. The program may be in the form of source code, object code, a code intermediate source and object code such as in partially compiled form, or in any other form suitable for use in the implementation of the process according to the invention. The program may either be a part of an operating system, or be a separate application. The carrier may be any entity or device capable of carrying the program. For example, the carrier may comprise a storage medium, such as a Flash memory, a ROM (Read Only Memory), for example a DVD (Digital Video/ Versatile Disk), a CD (Compact Disc) or a semiconductor ROM, an EPROM (Erasable Programmable Read-Only Memory), an EEPROM (Electrically Erasable Programmable Read-Only Memory), or a magnetic recording medium, for example a floppy disc or hard disc. Further, the carrier may be a transmissible carrier such as an electrical or optical signal which may be conveyed via electrical or optical cable or by radio or by other means. When the program is embodied in a signal which may be conveyed directly by a cable or other device or means, the carrier may be constituted by such cable or device or means.

Alternatively, the carrier may be an integrated circuit in which the program is embedded, the integrated circuit being adapted for performing, or for use in the performance of, the relevant processes.

Variations to the disclosed embodiments can be understood and effected by those skilled in the art in practicing the claimed invention, from a study of the drawings, the disclosure, and the appended claims.

The term "comprises/comprising" when used in this specification is taken to specify the presence of stated features, integers, steps or components. The term does not preclude the presence or addition of one or more additional elements, features, integers, steps or components or groups thereof. The indefinite article "a" or "an" does not exclude a plurality. In the claims, the word "or" is not to be interpreted as an exclusive or (sometimes referred to as "XOR"). On the contrary, expressions such as "A or B" covers all the cases "A and not B", "B and not A" and "A and B", unless otherwise indicated. The mere fact that certain measures are recited in mutually different dependent claims does not indicate that a combination of these measures cannot be used to advantage. Any reference signs in the claims should not be construed as limiting the scope.

It is also to be noted that features from the various embodiments described herein may freely be combined, unless it is explicitly stated that such a combination would be unsuitable.

The invention is not restricted to the described embodiments in the figures but may be varied freely within the scope of the claims.

## Claims

1. A method for making medical data available to third parties, which method is performed in at least one processor (160) and comprises:
obtaining, via a first interface (110), a digital storage agreement (R[auth]) from a terminal (UT), which digital storage agreement (R[auth]) authorizes storage of medical data (PD_{ID}) in a central database (140), which medical data relates to a user of the terminal (UT);
sending, via a second interface (120), in response to the digital storage agreement (R[auth]), a first data request (RQ1) to a primary server (JS), which first data request (RQ1) is configured to cause the primary server (JS) to forward medical data (PD_{ID}) relating the user from the primary server (JS) to the central database (140),
obtaining medical data (PD_{ID}) relating to the user via the second interface (120);
storing the obtained medical data (PD_{ID}) in the central database (140);
receiving, via a third interface (130), a data enquiry (ENQ) from a third party (DU), which data enquiry (ENQ) encompasses a request for the medical data (PD_{ID}) relating to the user and which medical data (PD_{ID}) are stored in the central database (140);
checking (CHK2), in response to the data enquiry (ENQ), if the user has authorized sharing (ACC) the medical data (PD_{ID}) requested in the data enquiry (ENQ) with the third party (DU), and only if the user has authorized said sharing;
forwarding a copy of the medical data (PD_{ID}) requested in the data enquiry (ENQ) to the third party (DU) via the third interface (130).

2. The method according to claim 1, comprising:
receiving, via the first interface (110), a digital sharing authorization (ACC) from the terminal (UT), which digital sharing authorization is configured to authorize the sharing of the medical data (PD_{ID}) requested in the data enquiry (ENQ) with the third party (DU); and
storing the digital sharing authorization (ACC) in a contract database (150).

3. The method according to any one of the claims 1 or 2, wherein the checking (CHK2) if the user has authorized sharing the medical data (PD_{ID}) requested in the data enquiry (ENQ) with the third party (DU) comprises:
searching the contract database (150) for the digital sharing authorization (ACC); and
allowing forwarding of the copy of the medical data (PD_{ID}) requested in the data enquiry (ENQ) to the third party (DU) via the third interface (130) exclusively if the digital sharing authorization (ACC) is found in the contract database (150).

4. The method according to any one of the claims 2 or 3, wherein the digital sharing authorization (ACC) defines one of:
a subset, or
a complete amount
of the medical data (PD_{ID}) relating to the user stored in the central database (140).

5. The method according to any one of the claims 2 to 4, wherein the digital sharing authorization (ACC) has a time limit after which it expires and ceases to be valid.

6. The method according to any one of the preceding claims, wherein the digital storage agreement (R[auth]) defines one of:
a subset, or
a complete amount
of medical data (PD_{ID}) relating to the user stored in a primary database (JDB) controlled by the primary server (JS).

7. The method according to any one of the preceding claims, wherein the digital sharing authorization (ACC) has a time limit after which it expires and ceases to be valid.

8. A computer program product (175) loadable into a non-volatile data carrier (170) communicatively connected to at least one processor (160), the computer program product (175) comprising software configured to, when the computer program product (175) is run on the at least one processor (160), cause the at least one processor (160) to perform the method of any of the claims 1 to 7.

9. A non-volatile data carrier (170) containing the computer program product of the claim 8.

10. A server (100) for making medical data available to third parties, the server (100) comprising:
a first interface (110) configured to obtain a digital storage agreement (R[auth]) from a terminal (UT), which digital storage agreement (R[auth]) authorizes storage of medical data (PD_{ID}) relating to a user of the terminal (UT) in a central database (140) communicatively connected to the server (100);
a second interface (120) configured to:
send, in response to the digital storage agreement (R[auth]), a first data request (RQ1) to a primary server (JS), which first data request (RQ1) is configured to cause the primary server (JS) to forward medical data (PD_{ID}) relating to the user to the second interface (120), and
obtain medical data (PD_{ID}) relating to the user from the primary server (JS);
wherein the server (100) is configured to store the obtained medical data (PD_{ID}) in the central database (140);
a third interface (130) configured to receive a data enquiry (ENQ) from a third party (DU), which data enquiry encompasses a request for the medical data (PD_{ID}) relating to the user stored in the central database (140);
wherein the server (100) is further configured to:
check, in response to the data enquiry (ENQ), if the user has authorized (ACC) sharing the medical data (PD_{ID}) requested in the data enquiry (ENQ) with the third party (DU), and only if the user has authorized (ACC) said sharing;
forward a copy of the medical data (PD_{ID}) requested in the data enquiry (ENQ) to the third party (DU).

11. The server (100) according to claim 10, wherein:
the first interface (110) is configured to receive a digital sharing authorization (ACC) from the terminal (UT), which digital sharing authorization is configured to authorize the sharing of the medical data (PD_{ID}) requested in the data enquiry (ENQ) with the third party (DU); and
the server is configured to store the digital sharing authorization (ACC) in a contract database (150).

12. The server (100) according to any one of the claims 10 or 11, being further configured to check (CHK2) if the user has authorized sharing the medical data (PD_{ID}) requested in the data enquiry (ENQ) with the third party (DU) by:
searching the contract database (150) for the digital sharing authorization (ACC); and
allowing forwarding of the copy of the medical data (PD_{ID}) requested in the data enquiry (ENQ) to the third party (DU) via the third interface (130) exclusively if the digital sharing authorization (ACC) is found in the contract database (150).

13. The server (100) according to any one of the claims 11 or 12, wherein the digital sharing authorization (ACC) defines one of:
a subset, or
a complete amount of the medical data (PD_{ID}) relating to the user stored in the central database (140).

14. The server (100) according to any one of the claims 11 to 13, wherein the digital sharing authorization (ACC) has a time limit after which it expires and ceases to be valid.

15. The server (100) according to any one of the claims 10 to 14, wherein the digital storage agreement (R[auth]) defines one of:
a subset, or
a complete amount of medical data (PD_{ID}) relating to the user stored in a primary database (JDB) controlled by the primary server (JS).

16. The server (100) according to any one of the claims 10 to 15, wherein the digital sharing authorization (ACC) has a time limit after which it expires and ceases to be valid.
